Europäisches Patentamt

European Patent Office

Office européen des brevets

(19)

(11) Numéro de publication: **0 261 038 B1**

# FASCICULE DE BREVET EUROPEEN

(12)

(45) Date de publication de fascicule du brevet:
30.10.91

(51) Int. Cl.⁵: **A61B 17/58**

(21) Numéro de dépôt: 87420209.6

(22) Date de dépôt: 30.07.87

(54) **Agrafe de fixation, notamment pour ostéosynthèse et son procédé de fabrication.**

(30) Priorité: 18.08.86 FR 8612052

(43) Date de publication de la demande:
23.03.88 Bulletin 88/12

(45) Mention de la délivrance du brevet:
30.10.91 Bulletin 91/44

(84) Etats contractants désignés:
AT BE CH DE ES FR GB GR IT LI LU NL SE

(56) Documents cités:
FR-A- 2 231 189
US-A- 1 547 862
US-A- 3 166 072
US-A- 3 586 002
US-A- 3 939 828

(73) Titulaire: SILAB S.A.R.L.
20 Boulevard Galliéni
F-92130 ISSY LES MOULINEAUX(FR)

(72) Inventeur: Gauthier, Georges
85, cours Albert-Thomas
F-69003 Lyon(FR)

(74) Mandataire: Maureau, Pierre et al
Cabinet GERMAIN & MAUREAU B.P. 3011
F-69392 Lyon Cédex 03(FR)

**Description**

La présente invention a pour objet une agrafe de fixation, notamment pour ostéosynthèse, et son procédé de fabrication, c'est-à-dire une agrafe tout particulièrement utilisable comme moyen de fixation dans les matériaux relativement peu résistants comme le bois ou ses dérivés, les matériaux synthétiques plastiques, ou pour la fixation osseuse dite ostéosynthèse.

Il est admis que la consolidation osseuse d'une fracture est impossible ou quasiment impossible s'il existe des mouvements d'amplitude notable dans le sens du cisaillement au niveau de la surface fracturaire. En revanche, la consolidation se fait d'une façon d'autant plus favorable que sont appliquées des contraintes de pression variables et non excessives, perpendiculaires à la fracture. Or, les moyens d'ostéosynthèse connus et couramment utilisés sont loin de réaliser une fixation osseuse idéale interdisant les cisaillement et favorisant des contraintes de pression variables et non excessives perpendiculaires à la fracture.

On peut très schématiquement opposer deux groupes de moyens connus de fixation osseuse : les moyens de fixation rigide ou ostéosynthèse lourde et les moyens de fixation souple ou ostéosynthèse légère. On peut qualifier d'ostéosynthèse lourde les moyens de fixation constitués par un matériel volumineux comme les plaques vissées ou les enclouages après alésage. Ces moyens de fixation fournissent des immobilisation de la zone fracturaire tout à fait rigides. Cette rigidité évite bien le cisaillement de la zone fracturaire qui se consolide dans la quasi totalité des cas, mais cette rigidité ne permet pas, ou peu, les pressions variables perpendiculaires à la zone fracturaire favorables à la consolidation, de sorte que cette dernière se fait souvent avec des durées prolongées, voire très prolongées, par rapport aux possibilités optimales de consolidation osseuse. De plus, l'utilisation de ces moyens oblige à dévitaliser peu ou prou les tissus, et tout spécialement l'os, ce qui entraîne des aléas dont le plus important est le risque de contamination septique extensive, parfois grave, et souvent difficile à guérir. En outre, l'ablation du matériel d'ostéosynthèse est en principe nécessaire ; elle se fait en général environ dix-huit mois après la mise en place, pour éviter les modifications durables de la physiologie osseuse induite par l'importance de la rigidité du matériel, constituées de spongialisation ou d'ostéoporose extensive de l'os ainsi déchargé de sa fonction physiologique.

Les moyens d'ostéosynthèse légère comprennent les petites broches et les agrafes classiques dont certaines comportent deux tiges latérales sensiblement parallèles, reliées entre elles par une partie centrale rectiligne ou angulée, formant pont, et dont l'axe médian est situé dans le même plan que les axes médians des tiges latérales. Ces moyens connus d'ostéosynthèse légère présentent l'avantage de laisser se manifester, au niveau du foyer fracturaire, des compressions induites par les contractions des muscles squelettiques du membre. Malheureusement, à eux seuls, ils n'interdisent pas toujours de façon très satisfaisante les mouvements de cisaillement particulièrement défavorables à la consolidation et, lorsqu'ils sont employés seuls, ils donnent des risques de pseudarthrose ; lorsqu'on leur adjoint des moyens de contention complémentaire, comme un plâtre, cela se fait au détriment du confort de l'opéré et ne peut constituer, en régle générale, une immobilisation idéale des zones fracturaires dans le sens du cisaillement.

L'agrafe selon l'invention vise à permettre une fixation nettement plus satisfaisante des éléments osseux en contact car plus proche des conditions optimales de la consolidation osseuse tout en évitant, par cette ostéosynthèse simple, toute dévitalisation. Grâce à sa conception et à une mise en place adaptée au type d'ostéosynthèse, cette agrafe évite tout cisaillement, tout en permettant des compressions variables perpendiculaires à la zone fracturaire.

Dans cette agrafe qui est du type précité comportant deux tiges latérales parallèles reliées entre elles à leurs extrémités proximales par une partie centrale formant pont, la partie centrale forme une courbe unique plane, sans point d'inflexion, de telle sorte que son axe médian soit contenu dans un plan, et est disposée de manière que ce plan forme un angle avec le plan contenant les axes longitudinaux des deux tiges. Cet angle de pliage est supérieur à 90°.

Suivant une forme d'exécution préférée de l'invention, les tiges latérales de cette agrafe sont de section circulaire et de longueurs inégales, ce qui facilite, respectivement, la réalisation des trous pour les recevoir et leur introduction dans ces trous.

Pour faciliter le guidage des tiges latérales et diminuer les risques de les déformer, lors de la mise en place de l'agrafe, la partie distale, ou extrémité libre de tige latérale, est avantageusement d'un diamètre légèrement inférieur à celui du reste de chaque tige et, de préférence, cette partie distale est polie pour faciliter son glissement dans le trou préalablement foré dans l'os.

Cette agrafe est de préférence réalisée en un matériau biocompatible et présentant une bonne élasticité et une bonne résistance mécanique, par exemple un métal tel qu'un alliage de titane comme le TA6V de qualité médicale.

Suivant un mode de mise en oeuvre simple de l'invention, le procédé de fabrication de cette agrafe consiste à conformer un fil du matériau désiré en épingle à cheveux, avec une partie centrale de liaison curviligne, puis à rabattre cette partie centrale en direction des deux branches latérales, sur un angle supérieur à 90°, et selon une forme de courbure prédéterminée ne comportant pas d'angle vif.

Il résulte de la forme de cette agrafe de nouvelles possibilités utilisant bien les qualités de plasticité et d'élasticité de son matériau constitutif.

Cette agrafe en forme d'épingle à cheveux coudée permet, sur la partie proximale réunissant les deux branches, une mise en place avec un appui sur la surface du matériau à fixer par sa partie centrale repliée. Le fait que le métal de l'agrafe soit modelable, aide à une adaptation exacte et plusieurs applications pratiques peuvent être envisagées. Le fait que le métal présente une élasticité permet des micro-mouvements.

De toute façon, l'invention sera bien comprise à l'aide de la description qui suit, en référence au dessin schématique annexé représentant une forme d'exécution de cette agrafe et plusieurs exemples de son utilisation en ostéosynthèse :

Figures 1 et 2 sont des vues, respectivement, en perspective et de côté en élévation de l'agrafe selon l'invention ;

Figure 3 est une vue en plan par-dessus du squelette d'un pied gauche montrant l'application de cette agrafe à une ostéosynthèse pour arthrodèse de l'articulation métatarso-phalangienne du gros orteil de ce pied ;

Figures 4 et 5 illustrent un mode d'utilisation de cette agrafe pour la correction d'une déviation de la partie distale du tibia ;

Figures 6 et 7 illustrent un troisième mode d'utilisation de cette agrafe à la correction d'une malformation du calcanéum.

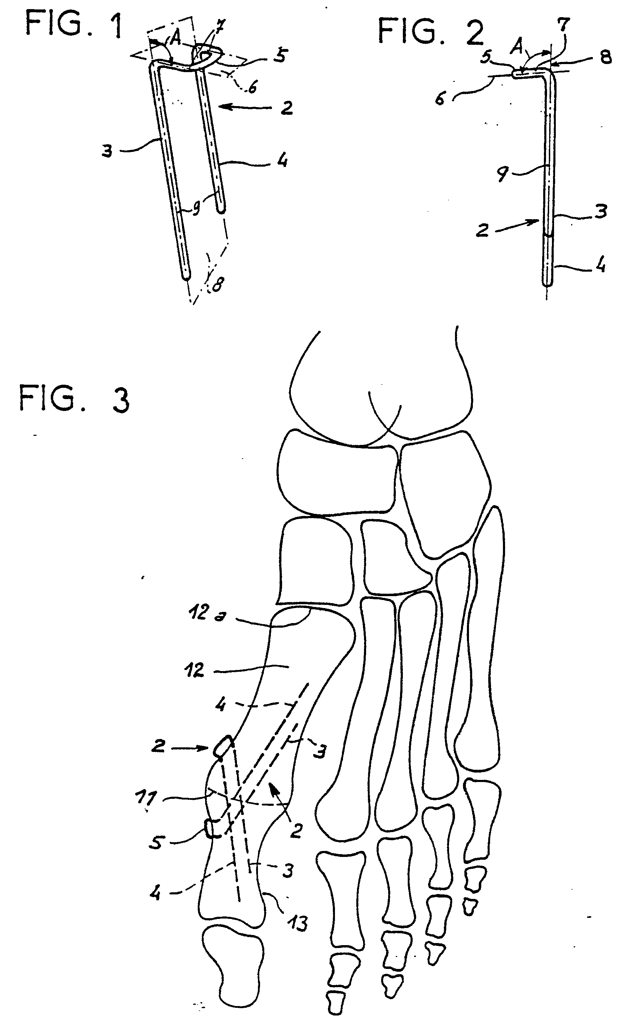

Comme le montrent les figures 1 et 2, cette agrafe 2 est du type comportant deux tiges latérales 3 et 4 sensiblement parallèles reliées entre elles à leurs extrémités proximales par une partie centrale 5 formant pont. Selon l'invention, la partie centrale formant pont 5 est curviligne, plane, et, en outre, elle est disposée de manière que l'angle formé par le demi-plan contenant la courbe plane et le demi-plan contenant les extrémités distales des tiges est au plus égal à 90°.

Suivant une forme d'exécution intéressante de l'invention, les tiges latérales 3 et 4 de cette agrafe 2 sont de section circulaire, ce qui facilite la réalisation des trous forés pour les recevoir dans les parties d'os à la tenue desquelles elle doivent participer. En outre, pour faciliter leur engagement dans ces trous, les tiges 3 et 4 sont de longueurs inégales. Dans l'exemple illustré sur le dessin, la tige 4 est plus longue que la tige 3.

Les trous dans lesquels les tiges latérales 3 et 4 de cette agrafe doivent être engagées sont forés au même diamètre que les tiges, de telle sorte que leur engagement dans ces trous, notamment si elles sont de grande longueur, peut rencontrer une résistance due aux forces de frottement ou de coincement. Il peut en résulter une déformation accidentelle et indésirable telle qu'un pliage, de l'une ou des deux tiges.

Pour y remédier, la partie distale ou extrémité libre de chaque tige latérale 3, 4 est d'un diamètre légèrement inférieur à celui du reste de la tige. Cette extrémité plus fine de chaque tige 3, 4 assure son rôle de guidage de la tige considérée de l'agrafe lors de son engagement dans le trou foré pour la recevoir, sans risque de coincement.

Pour faciliter encore mieux cet engagement, chaque extrémité affinée des tiges latérales 3, 4 de cette agrafe est polie de manière à mieux glisser dans son trou.

Naturellement, cette agrafe 2 doit être réalisée en un matériau biocompatible et présenter tout à la fois une bonne élasticité et une bonne résistance mécanique. Un métal comme un alliage de titane tel que le TA6V de qualité médicale convient parfaitement.

Un procédé très simple de fabrication de cette agrafe consiste à conformer le fil de son matériau constitutif en épingle à cheveux, avec une partie centrale 5, formant pont, de forme curviligne plane, puis à rabattre cette partie centrale 5 en direction des deux branches latérales 3 et 4 sur un angle A supérieur à 90°, cette pliure étant exécutée de façon à éviter la détérioration des qualités mécaniques du matériau. Il faut, en particulier, une instrumentation permettant une courbure régulière, et non un angle vif facteur d'amorce de rupture. De plus, en fonction du matériau constitutif, il est bon de conformer l'agrafe dans les conditions qui évitent écrouissage ou modifications des qualités mécaniques, par exemple à chaud.

La figure 3 illustre l'application de cette agrafe à une ostéosynthèse pour arthrodèse de l'articulation métatarso-phalangienne du gros orteil d'un pied gauche. Dans cette application, chaque agrafe 2 est utilisée en ostéosynthèse longitudinale, c'est-à-dire que leurs branches 3 et 4 sont engagées dans l'os suivant une direction voisine de celle de l'axe longitudinale de celle de l'os considéré, la partie 5 formant pont de chaque agrafe 2 étant amenée en appui cortical dans l'os épiphysaire.

Il faut noter que l'arthrodèse de la première articulation métatarso-phalangienne présente, selon certaines statistiques, un pourcentage très élevé de non consolidation lorsqu'on utilise des moyens traditionnels de fixation tels que par vis. Selon l'invention, cette fixation est réalisée à l'aide d'au moins une agrafe 2 dont les deux tiges 3 et 4 sont engagées sensiblement perpendiculairement au plan 11 de soudre osseuse et interdisent donc tout mouvement de cisaillement, aussi bien en translation qu'en rotation. Si l'on n'utilise qu'une seule agrafe 2, ses tiges doivent être engagées à l'intérieur du corps du premier métatarsien 12, de manière à arriver à proximité de sa base 12a, la partie centrale coudée 5 de cette agrafe étant en appui sur la corticale de la phalange 13 du gros orteil. Cette partie centrale coudée 5 permet, par percussion, une coaptation de la zone fracturaire 11 à laquelle contribue la fixation des tiges 3 et 4 de l'agrafe 2 qui interdisent son arrachement. Par contre, lors des contractions musculaires, des pressions va riables favorables à la consolidation peuvent se produire au niveau de cette zone fracturaire.

Comme le montre la figure 3, il est possible d'utiliser une seconde agrafe 2 dont les tiges 3 et 4 sont engagées dans le corps de la première phalange 13 de ce gros orteil.

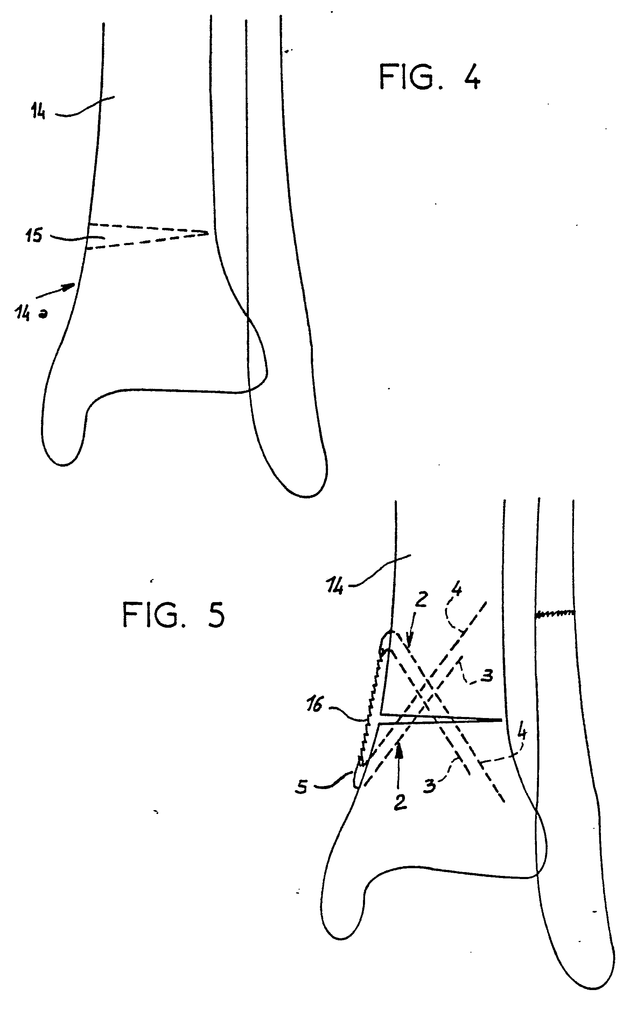

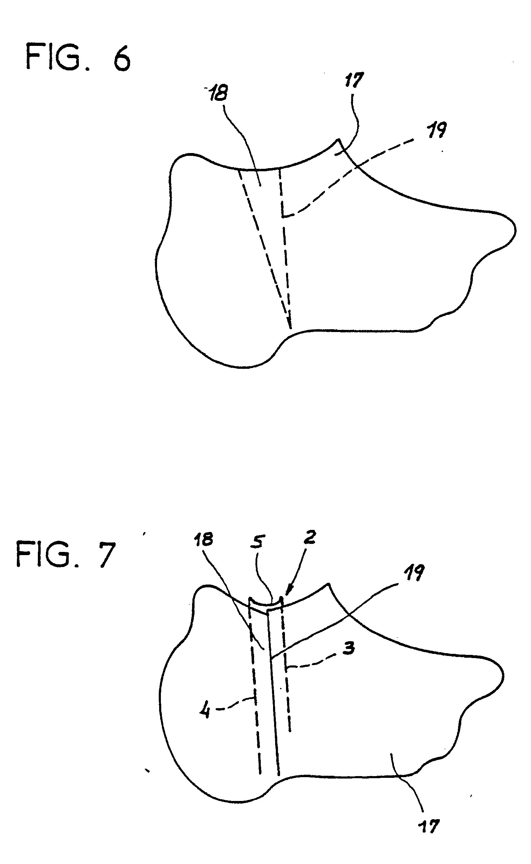

Les figures 4 et 5 illustrent un autre mode d'utilisation de cette agrafe, dans le cas de son application à une fixation de la partie distale 14a du tibia 14, après correction d'une déviation de ce tibia par résection d'une partie 15 en forme de coin, visible sur la figure 4. Après résection de cette partie 15 du tibia 14, deux agrafes 2 sont implantées des le tibia 14 de manière à ce que chacune d'elles traverse le plan de section des deux parties de ce tibia à coapter. Comme dans l'application précédente, les deux agrafes 2 interdisent tout déplacement par rotation ou par translation des deux surfaces en contact.

Comme le montre la figure 5, dans cette application, la coaptation des surfaces en contact peut être améliorée en utilisant les parties coudées 5 des deux agrafes 2 comme tête d'amarrage pour un organe tendeur ou ressort 16 qui peut être soit en métal de même nature que les agrafes 2, soit en un autre matériau biocompatible et, notamment, en un matériau biodégradable comme le polyglycol.

Les figures 6 et 7 montrent une autre application de cette agrafe 2 dans laquelle la composition des deux parties à consolider d'un os est obtenue en mettant à profit l'élasticité de la partie coudée 5 de l'agrafe. Dans l'exemple illustré par les figures 6 et 7, l'agrafe 2 est utilisée comme moyen de rappel élastique des deux surfaces à souder d'un calcanéum 17 ayant préalablement subi une correction par résection. La figure 6 montre ce calcanéum 17 avant correction par résection d'une partie 18 en forme de coin.

Comme le montre la figure 7, dans cette application, l'élasticité de la partie centrale coudée 5 de l'agrafe 2 est mise à profit pour assurer la pression de contact des deux surfaces de la zone fracturaire 19.

Il a été indiqué précédemment que les agrafes 2 étaient avantageusement en alliage de titane TA6V de qualité médicale. Non seulement cet alliage présente une caractéristique tout à fait remarquable de biocompatibilité absolue et constante mais, en outre, il possède des qualités de résistance mécanique très supérieure aux aciers habituellement utilisés en ostéosynthèse, et plus particulièrement il possède une excellente résistance à la fatigue. Il présente enfin l'avantage d'adhérer facilement à l'os et de ne provoquer aucune réaction de ce dernier à son contact.

Il faut cependant noter que son usinage est particulièrement difficile et que, pour obtenir ses qualités optimales de résistance mécanique, il doit être travaillé à des températures de l'ordre de 950° à 1 000° et sous atmosphère d'argon. On comprend aisément que, dans ces conditions, un usinage de pièce complexe entraînerait des coûts importants compte tenu, d'une part, du prix du matériau et, d'autre part, de la complexité de sa technologie de transformation. Par contre, la forme simple de l'agrafe de l'invention maintient son prix de revient à un niveau tout à fait raisonnable.

Il a été indiqué précédemment que les tiges 3 et 4 cette agrafe 2 étaient avantageusement de longueurs inégales. Naturellement, il est possible de fabriquer chaque agrafe 2 avec des tiges 3 et 4 de même longueur, le praticien pouvant sectionner les deux branches aux longueurs désirées au moment de l'intervention.

On comprendra aisément que, lors de cette intervention, les perforations réalisées dans l'os distal pour l'engagement des tiges 3 et 4 de chaque agrafe 2 sont avantageusement de section transversale légèrement inférieure à celle des tiges 3 et 4 de l'agrafe 2 considérée pour assurer leur bon ancrage dans cet os tandis que les perforations réalisées dans l'os proximal sont avantageusement de section transversale égale ou légèrement supérieure à celle des tige s 3 et 4, de manière à favoriser leur glissement.

## Revendications

1. Agrafe de fixation, notamment pour ostéosynthèse, comportant deux tiges latérales (3,4) parallèles, reliées entre elles à leurs extrémités proximales par une partie centrale (5), formant pont, caractérisée en ce que la partie centrale (5) forme une courbe unique plane sans point d'inflexion et que la tête de l'agrafe constituée par la partie centrale (5) est repliée ; l'angle de pliage (A) est tel que l'angle formé par le demi-plan contenant la courbe plane et le demi-plan contenant les extrémités distales des tiges (3,4) est au plus égal à 90°.

2. Agrafe selon la revendication 1, caractérisée en ce que ses tiges latérales (3,4) sont de section circulaire.

3. Agrafe selon l'une quelconque des revendications précédentes, caractérisée en ce que ses tiges latérales (3,4) sont de longueurs inégales.

4. Agrafe selon l'une quelconque des revendications précédentes, caractérisée en ce que la partie distale ou extrémité libre de chaque tige latérale (3,4) présente un diamètre très légèrement inférieur à celui du reste de la tige considérée.

5. Agrafe selon la revendication 4, caractérisée en ce que la partie distale ou extrémité libre de chaque tige latérale (3,4) de plus faible diamètre que le reste de la tige présente un état de suface poli.

6. Agrafe selon l'une quelconque des revendications précédentes, caractérisée en ce qu'elle est réalisée en un matériau biocompatible et présentant une bonne élasticité et un bonne résistance mécanique.

7. Agrafe selon la revendication 6, caractérisée en ce qu'elle est réalisée en métal tel qu'un alliage de titane de qualité médicale.

8. Procédé de fabrication de l'agrafe selon l'une quelconque des revendications précédentes, caractérisé en ce qu'il consiste à conformer un fil du matériau désiré en épingle à cheveux, avec une partie centrale de liaison (5) curviligne et plane, puis à rabattre cette partie centrale (5) en direction des deux branches latérales (3,4) sur un angle (A) supérieur à 90°, en conservant les qualités mécaniques du matériau par le contrôle de la forme exacte des courbures, sans angle vif, et les conditions en particulier thermiques dans lesquelles sont effectuées les opérations de conformation.

## Claims

1. Fixing staple, in particular for osteosynthesis, having two parallel lateral rods (3, 4) connected to one another at their proximal ends by a central part (5) forming a bridge, characterized in that the central part (5) forms a single planar curve without a reversal point, and in that the head of the staple formed by the central part (5) is folded back; the angular folding (A) is such that the angle formed by the half plane containing the planar curve and a half plane containing the distal ends of the rods (3, 4) is at most equal to 90°.

2. Staple according to Claim 1, characterized in that its lateral rods (3, 4) are circular in section.

3. Staple according to either of the preceding claims, characterized in that its lateral rods (3, 4) are unequal in length.

4. Staple according to any one of the preceding claims, characterized in that the distal part or free end of each lateral rod (3, 4) has a diameter very slightly smaller than that of the rest of the rod concerned.

5. Staple according to Claim 4, characterized in that the distal part or free end of each lateral rod (3, 4) of smaller diameter than the rest of the rod has a polished surface finish.

6. Staple according to any one of the preceding claims, characterized in that it is made of a biocompatible material having good resilience and good mechanical strength.

7. Staple acccrding to Claim 6, characterized in that it is made of metal, such as a titanium alloy, of medical-grade quality.

8. Process for manufacturing the staple according to any one of the preceding claims, characterized in that it consists in shaping a wire of the desired material into a hairpin, with a curved planar central connecting part (5), then in turning down this central part (5) in the direction of the two lateral legs (3, 4) to an angle (A) greater than 90°, while maintaining the mechanical qualities of the material by controlling the exact shape of the curves, without an acute angle, and the conditions in particular the thermal ones, in which the shaping operations are carried out.

## Patentansprüche

1. Befestigungsklammer, insbesondere für Osteosynthese, mit zwei parallelen seitlichen Stiften (3,4), die an ihren proximalen Enden durch eine brückenbildende Zentralpartie (5) miteinander verbunden sind, dadurch gekennzeichnet, daß die Zentralpartie (5) einen ebenen einzigen Bogen ohne Umkehrpunkt bildet und daß der von der Zentralpartie (5) gebildete Kopf der Klammer umgebogen ist, wobei der Biegewinkel (A) so ist, daß der durch die den ebenen Bogen enthaltende Halbebene und die die distalen Enden der Stifte (3,4) enthaltende Halbebene gebildete Winkel höchstens gleich 90° ist.

2. Klammer nach Anspruch 1, dadurch gekennzeichnet, daß ihre seitlichen Stifte (3,4) kreisförmigen Querschnitt aufweisen.

3. Klammer nach einem der vorstehenden Ansprüche, dadurch gekennzeichnet, daß ihre seitlichen Stifte (3,4) ungleich lang sind.

4. Klammer nach einem der vorstehenden Ansprüche, dadurch gekennzeichnet, daß die distale Partie oder freies Ende jedes seitlichen Stiftes (3,4) einen Durchmesser aufweist, der uni einen sehr geringen Betrag kleiner als der des restlichen Stiftes ist.

5. Klammer nach Anspruch 4, dadurch gekennzeichnet, daß die distale Partie oder freies Ende jedes seitlichen Stiftes (3,4) mit geringerem Durchmesser als der restliche Stift eine polierte Oberflächenbeschaffenheit aufweist.

6. Klammer nach einem der vorstehenden Ansprüche, dadurch gekennzeichnet, daß sie aus einem biologisch verträglichen Material besteht und eine gute Elastizität und einen guten mechanischen Widerstand aufweist.

7. Klammer nach Anspruch 6, dadurch gekennzeichnet, daß sie aus Metall wie eine Titanlegierung medizinischer Qualität besteht.

8. Verfahren zur Herstellung der Klammer gemäß einem der vorstehenden Ansprüche, dadurch gekennzeichnet, daß man einen Draht des gewünschten Materials zu einer Haarnadelgestalt mit einer gekrümmten und ebenen Verbindungs-Zentralpartie (5) formt, wonach man diese Zentralpartie (5) in Richtung der beiden seitlichen Stifte (3,4) um einen größeren Winkel als 90° umbiegt, wobei die mechanischen Eigenschaften des Materials durch Kontrollieren der exakten Bogenform, ohne scharfen Winkel, und der insbesondere thermischen Bedingungen, unter denen die Formgebung abläuft, beibehalten werden.

FIG. 1

FIG. 2

FIG. 3

FIG. 4

FIG. 5

FIG. 6

FIG. 7